(19) **Europäisches Patentamt / European Patent Office / Office européen des brevets**

(11) **EP 4 574 018 A1**

(12) **EUROPEAN PATENT APPLICATION**

(43) Date of publication:
**25.06.2025 Bulletin 2025/26**

(21) Application number: **23461699.3**

(22) Date of filing: **22.12.2023**

(51) International Patent Classification (IPC):
**A61B 3/11** $^{(2006.01)}$ **A61B 5/16** $^{(2006.01)}$
**A61B 5/00** $^{(2006.01)}$ **G16H 50/20** $^{(2018.01)}$
**G16H 50/70** $^{(2018.01)}$

(52) Cooperative Patent Classification (CPC):
**A61B 3/112; A61B 3/0025; A61B 5/163; A61B 5/4076; A61B 5/7267; G16H 50/20; G16H 50/70**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Solvemed Group sp. z o.o.**
**61-806 Poznan (PL)**

(72) Inventors:
• **BOGUCKI, Aleksander**
  **01-003 Warszawa (PL)**
• **CHRAPKIEWICZ, Radoslaw**
  **Sunnyvale, CA 94087 (US)**
• **CHROST, Hugo**
  **64-100 Leszno (PL)**
• **JACHURA, Michal**
  **02-353 Warszawa (PL)**
• **JOHN, Ivo**
  **00-514 Warszawa (PL)**
• **MANOHAR, Sanjay**
  **Oxford, OX3 9DU (GB)**
• **WLODARSKI, Michal**
  **85-829 Bydgoszcz (PL)**
• **ZINKIEWICZ, Lukasz**
  **02-881 Warszawa (PL)**

(74) Representative: **Cooley (UK) LLP**
**22 Bishopsgate**
**London EC2N 4BQ (GB)**

(54) **A METHOD FOR TRAINING A MACHINE LEARNING MODEL FOR AUTOMATICALLY DIAGNOSING ABNORMAL PUPIL BEHAVIOUR BASED ON ASSESSING PUPIL REACTIVITY INVULNERABLE TO EXTERNAL LIGHT CONDITIONS, A COMPUTER PROGRAM COMPRISING INSTRUCTIONS THEREOF, AND A SYSTEM IMPLEMENTING THE MODEL**

(57) A present inventions describe a method for training a machine learning model for automatically diagnosing abnormal pupil behaviour based on assessing pupil reactivity invulnerable to external light conditions, a computer program comprising instructions thereof, and a system implementing the model.

Fig. 7

EP 4 574 018 A1

## Description

### Technical field

The present disclosure relates to the field of medicine, ophthalmology, neuro-ophthalmology with a subfield of pupillometry. The subject matter of the invention relates to a method of assessing pupil reactivity insusceptible to external ilumination conditions, a method of performing pupil reactivity measurements and a method for assessing neurological health of a subject.

### Prior art

Typical pupillary evaluation during the medical examination involves the assessment of two components -pupil size and pupil reactivity to light. Pupil size is traditionally measured using a pupil gauge to estimate the diameter in millimeters of the pupil at rest state before any light is shone into the eye. Manual measurement is subjective and prone to subjective error of a medical personel performing the examination. Pupil light reactivity is evaluated by shining a light into a patient's eye to make the pupil constrict in reaction to the light. The pupil should dilate (expand its diameter) again when the light is moved away or turned off. One of the most frequently used oculography index is so-called Nuerological Pupil Index™ (or simply NPi) : [https.-//en.wikipedia.org/wiki/Neurological pupil index]. The pupil's reaction is numerically graded, typically using a range spanning the range 1-3 i.e. reactive, sluggish, non-reactive (these terms are subjective and applied without a standard clinical protocol or definition) or 0-4.9 as in a case of NPi.

In the paper, Ghauri, Muhammad S., et al. [Evaluating the reliability of neurological pupillary index as a prognostic measurement of neurological function in critical care patients." Cureus 14.9 (2022)] authors study the use of the Neurological Pupil index (NPi) in a clinical context. It found a statistically significant correlation between NPi and established neurological assessment tools like the Glasgow Coma Scale (GCS) and the modified Rankin Scale (mRS). Despite limitations such as the study's single-institutional nature, the paper suggests NPi can be a useful prognostic tool in neurocritical care settings.

As elucidated in scientific article published [Ong C, Hutch M, Smirnakis S. The Effect of Ambient Light Conditions on Quantitative Pupillometry. Neurocrit Care. 2019 Apr;30(2):316-321. doi: 10.1007/s12028-018-0607-8. PMID: 30218349.] automated devices collecting quantitative measurements of pupil size and reactivity are increasingly used for critically ill patients with neurological disease. The data presented in the publication show that ambient light levels impact pupil reaction parameters in both healthy and critically ill subjects and recommend that medical and clinical practitioners should standardize external lighting conditions in the medical premises to maximize measurement reliability.

In scientific publication [W. Taylor et al., entitled "Quantitative pupillometry, a new technology: normative data and preliminary observations in patients with acute head injury. Technical note.", JOURNAL OF NEUROSURGERY, 2003, (200301), vol. 98, no. 1] authors conclude that quantitative pupillometry is a reliable and safe method that provides detailed and accurate information regarding patterns of pupillary responsiveness. The research data presented in that article is based on the assessment of several canonical parameters used in the pupillometry such as maximum aperture (INIT), minimum aperture (END), reduction in pupil diameter (DELTA), constriction velocity (MCV) and pupil reaction latency (LAT). The publication doesn't discuss the effect of ambient light level on the pupillary responsiveness as well as does not suggest any means to assess pupil reactivity measured under different illumination conditions.

A prior art patent document [WO2009137614A2] describes a use of scalar value to assess neurological status of a patient. It decribes a methods, systems and devices for determining whether a patient has an abnormally high level of intracranial pressure. The method includes a pupillometer to obtain pupillary response data from the patient. The method further includes a data analysis system comprising a microprocessor that is in communication with the pupillometer. The microprocessor includes an algorithm that converts the pupillary response data to a scalar value that is indicative of the patient's level of intracranial pressure. The microprocessor can be a stand-alone computer connected to the pupillometer or it can be integral with the pupillometer. The scalar value can be represented by a numerical value, graphical depiction, color, sound, or other visual or audio means that indicates a value. The scalar value indicates that the patient's pupillary response characteristics is within a normal range, an abnormal range, or that the pupil is nonresponsive.

Another prior art patent document [WO2023059325A1] entitled "Methods, systems, programs and devices to predict patient outcome using differential in pupillary index", describes a method of predicting the outcome of a hospitalized acute brain injury patient. The method includes using a pupilometer to take paired pupillary measurements of a left and right eye of the patient; using the pupilometer to determine neurological pupil index (NPI) of the left eye and display it on a display of the pupillometer; using the pupillometer to determine NPI of the right eye and display it on the display of the pupillometer; calculating a differential between NPI of the left eye and NPI of the right eye and display said NPI differential on the display of the pupillometer; and predicting an unfavorable health or neurological outcome if the NPIs of the left and right eyes indicate normal pupillary responses for each eye but the NPI differential meets or exceeds a given threshold value.

Another patent document [US20190159671] describes a computer program for performing a method, that involves recording images of a response of a left pupil to a stimulus thereby resulting in a first set of sequential images; recording images of a response of a right pupil to the same stimulus at the same time as the first images were recorded, thereby resulting in a second set of sequential images. Next, displaying simultaneously on the display the first and the second set of images, wherein the two sets of images are synchronized, and wherein a center of the left pupil of each image from the first set of sequential images is aligned with a center of the right pupil from the second set of sequential images on the display. Purpose of an invention is to diagnose anisocoria and asymmetry in pupilary response, because in some cases it might be symptoms of very serious and immediately life threatening conditions, hence it is important for medical practitioners to have tools for easy, quick and convenient ways to accurately detect, measure, and view in real-time differences in pupilary size and pupilary reaction to stimulus between the right and left pupils.

## Problem from the state of the art

Clinicians and medical personal routinely check the pupils of critically injured and ill patients to monitor their neurological status. However, manual pupil measurements (performed using a handheld penlight or ophthalmoscope) have been shown to be subjective, inaccurate, and not repeatable or consistent. Automated pupillometers are used to assess a variety of objective statical and dynamical pupillary light response variables including pupil size, pupil constriction velocity, constriction latency, and dilation velocity. The problem from the state of the art is the reliable assessment of pupil reactivity especially in a wide range of external light conditions which affect the pupillary light response variables. Another problem is a construction of a pupil reactivity index which could reliably assess the reactivity of the pupil in highly varying external light condition.

## Solution to the problem

In order to solve the problem of pupil reactivity index invulnerable to external light conditions a set of light corrected pupillometry parameters selected from the group of I NIT, END, CAMP, ACV, ADV, MCV, PDV, DELTA, FIN, LAT, FT75, DAMP (defined in the glossary) is calculated using the cohort average values as well as light corrected values based on the predictive models and light sensor settings readout from a diagnostic device. The predictive models are optimized with a use of dataset D1 consisting of pupillary light reaction measurements performed on the plurality of subjects for various external light conditions. Afterwards a reactivity index is constructed as a machine learning model realised in the form of linear combination of numeric constant and light corrected parameters with a particular set of coefficient values optimized (model training) to ensure external light invariance as well as high discriminability between two groups in dataset D2 consisting e.g. of the measurements of reactive and non-reactive pupils.

## Glossary and definitions of terms known in the state of the art and used in the patent specification

Throughout the present specification (both above and below the glossary section), the terms used have the following meanings described in the definitions below:

The term **"pupillary light response"** or **"pupillary light reflex"** is an automatic physiological response which optimizes light reaching the retina by controlling the pupil diameter in response to the intensity (luminance) of light that falls on the retinal ganglion cells of the retina. The contraction or dilation of the pupil is realized using the iris sphincter.

The term **"pupillogram"** denotes a characteristic curve reflecting pupillary light response which is created by plotting time against the pupil diameter before, during and after light stimulus.

The term **"INIT"** is a pupillometric parameter specifying the pupil diameter before the light stimulation. The parameter is usually expressed in millimetres.

The term **"END"** is a pupillometric parameter specyfing the smallest pupil diameter observed during the light stimulation. The parameter is usually expressed in millimetres.

The term **"CAMP"** is a pupillometric parameter describing the amplitude of pupil constriction induced by the stimulus light. The parameter is usually expressed in millimetres.

The term **"ACV"** is a pupillometric parameter describing the average velocity of pupil constriction induced by the stimulus light. The parameter is usually expressed in millimetres/second.

The term **"ADV"** is a pupillometric parameter describing the average velocity of pupil dilation induced by turning off the stimulus light. The parameter is usually expressed in millimetres/second.

The term **"MCV"** is a pupillometric parameter describing the maximum velocity of pupil constriction induced by the stimulus light. The parameter is usually expressed in millimetres/second.

The term **"PDV"** is a pupillometric parameter describing the maximal (peak) velocity of pupil dilation induced by turning off the stimulus light. The parameter is usually expressed in millimetres/second.

The term **"DELTA"** is a pupillometric parameter describing the relative constriction of the pupil. It is defined as CAMP/INIT. The parameter dimensionless and is usually expressed in percents.

The term **"FIN"** is a pupillometric parameter specifying the pupil diameter which is asymptotically reached by the pupil after the light stimulus is over.

The term **"LAT"** is a pupillometric parameter describing the time interval between the onset of light stimulation and the commencement of pupil constriction.

The term **"FT75"** is a pupillometric parameter describing a time interval required to recuperate 75% of the constriction amplitude post constriction termination.

The term **"DAMP"** is a pupillometric parameter describing the amplitude of pupil dilation after the stimulus light is turned off. The parameter is usually expressed in millimetres and might be calculated using a formula DAMP = FIN-END.

The term **"diagnostic device"** or alternatively **"measurement device"** means electronic device equipped with a central processing unit, a memory unit, a light sensor (camera chip, photodiode, photoreceptor etc), a light source and optical imaging system with adjustable focusing distance, which can capture an image. This includes a dedicated stand-alone device, a digital camera, portable video device, or a smartphone equipped with one or several cameras. The said optical imaging system with adjustable focusing distance contains a plurality of lenses.

The term **"visible light"** means a part of electromagnetic light spectrum which can be detected by the eye of the measured subject such as human or animal and thus induce pupillary light response.

The term **"exposure time - $\tau$"** means the setting of light sensor which defines the exposition time $\tau$ of each video frame. Usually, exposure time values ranging from below milliseconds up to tens of milliseconds are used.

The term **"dataset D1"** denotes a set of data consisting of pupillary light reaction measurements performed using a diagnostic device on the cohort of plurality of subjects for various external light conditions spanning the expected range of further external light invulnerability of reactivity assessment.

The term **"dataset D2"** denotes a set of data consisting of pupillary light reaction measurements divided into a pair of labeled groups i.e. the first group (control group) and the second group (experimental group). As an example it might consist of data consisting of pupillary light reaction measurements performed on the plurality of healthy subjects, with each subject subjected to the administration of drug reducing pupil reactivity (such as tropicamide or pilocarpine) and the reactivity measurements are performed before (these measurements are labeled as "pre") and after the drug administration (these measurements are labeled as "post").

The term **"exposure value"** also referred to as EV is an indicator of camera settings, corresponding to actual combination of exposure time, aperture size and ISO setting such that all combinations of aforementioned parameters that yield the same exposure conditions result in the same EV of the captured image. The term exposure value is of a broad use in photography.

The term **"external light"** means total light illuminating the pupil independently from stimulus light source. Sources of the external light might include room lighting, day light or any other source which is not controlled by the diagnostic device. Non-controlled environmental conditions such as changing intensity of external light affects the initial pupil diameter and in consequence increases the variability of pupillary light response in visible-light pupillometry performed by a diagnostic device.

The term **"ISOtau"** is an aggregated parameter read out from the camera settings of light sensor which corresponds to the external light illuminating the subject pupil. The parameter ISOtau is calculated by multiplying exposure value parameter ISO and exposure time parameter $\tau$.

The term **"focusing element"** means a moveable plurality of lenses (or lens assembly) or a single lens which is a part of camera imaging system and is responsible for setting the focusing distance of the camera.

The term "ISO" means the gain setting of light sensor which modifies its sensitivity to capturing light. Higher ISO values correspond to high sensor sensitivity allowing for video capture in low external light conditions, whereas lower ISO values correspond to low sensor sensitivity allowing for video capture in high external light conditions. The parameter is a digital equivalent of an exposure index used for the characterisation of analog photographic film.

The term **"light sensor"** means a CCD image sensor, EMCCD image sensor, CMOS image sensor, sCMOS sensor containing an array of light sensitive pixels used for creating digital images or other optoelectronic device such as photodiode or photoresistor.

The term **"region of interest"** is a selected part of an image which provides a basis for the exposure setting algorithm.

The term **"stimulus light"** means a light emitted from a visible light source that is generated by the diagnostic device in order to induce pupillary light reaction. A level of a stimulus light is controlled by the diagnostic device.

The term **"sigmoid function"** means any bounded, real function that is defined for all real input values and has a non-negative derivative at each point and exactly one inflection point. An example being logistic function, hyperbolic tangent, error function, spline or piecewise linear function.

The term **"$X^{mes}$"** denotes a set of raw pupillometry parameters extracted from measured pupillograms, a set of measured pupillometry parameters $X^{mes}$ comprising $X_1^{mes} = $ INIT, $X_2^{mes} = $ END, $X_3^{mes} = $ CAMP, $X_4^{mes} = $ ACV, $X_5^{mes} = $ ADV, $X_6^{mes} = $ MCV, $X_7^{mes} = $ PDV, $X_8^{mes} = $ DELTA, $X_9^{mes} = $ FIN, $X_{10}^{mes} = $ LAT, $X_{11}^{mes} = $ FT75, $X_{12}^{mes} = $ DAMP.

The term "$X^{corr}$" denotes a set of light corrected parameter values that are calculated using a method according to the invention.

The term "$X^{pred}$" denotes a set of models predicted parameters, each model responsible for predicting a behaviour of a single pupillometric parameter.

The term **"static pupillometric parameters"** or **"static parameters"** denotes a subset of parameters including: INIT, END, CAMP, DELTA, FIN, LAT, FT75, DAMP.

The term **"dynamic pupillometric parametes"** or **"dynamic parameters"** denotes a velocity based subset of parameters including: PDV, ACV, MCV, ADV.

## Summary of the invention

An invention relates to a method for training a machine learning model for automatically diagnosing abnormal pupil behaviour through assigning a numerical scale to pupil reactivity, comprising the following steps:

(a) a pupillary light reaction measurements are performed using a diagnostic device on the cohort of plurality of subjects under various external light conditions spanning the expected range of further external light invulnerability forming a first dataset (D1), similarly pupillary light reaction measurements are performed on the cohort of plurality of subjects with the measurements divided into a pair of labeled groups, with a first group of measurements performed before administration of drug reducing pupil reactivity (such as tropicamide or pilocarpine) and the second group of measurements performed after the drug administration, both forming a dataset (D2)

(b) based on the measured pupillograms, at least one parameter selected from a set of measured pupillometry parameters $\boldsymbol{X}^{mes}$ including $X_1^{mes}$ = INIT, $X_2^{mes}$ = END, $X_3^{mes}$ = CAMP, $X_4^{mes}$ = ACV, $X_5^{mes}$ = ADV, $X_6^{mes}$ = MCV, $X_7^{mes}$ = PDV, $X_8^{mes}$ = DELTA, $X_9^{mes}$ = FIN, $X_{10}^{mes}$ = LAT, $X_{11}^{mes}$ = FT75, $X_{12}^{mes}$ = DAMP , is extracted from each measurement;

(c) parameters of light sensor reflecting external light intensity such as the sensitivity ISO and/or exposure time $\tau$ are read out from a camera forming a single parameter ISOtau = ISO*$\tau$;

(d) for each pupillometry parameter $\boldsymbol{X_i}$ selected in step (b) (except for $X_1^{mes}$ =INIT ) its cohort average value is calculated based on the dataset D1, referred as $X_i^{avg}$ ;

(e) for each canonical pupillometry parameter $\boldsymbol{X_i}$ (except for $X_1^{mes}$ =INIT ) a multiple predictive models are created were each model is a linear combination of the predictors ISOtau, INIT, their logarithms, squares, cubes, reciprocals as well as the pairwise products of before-mentioned giving in total $\boldsymbol{N}$ predictors of a model, the predictive models are constructed using a binary feature inclusion with a total number of models equal $\boldsymbol{2^N}$ ;

(f) the selection of the best model from all created models in step (e) predicting a pupillometry parameter $\boldsymbol{X_i}$ relies on selecting the model with the lowest Bayesian information criterion (BIC) or similar method of model selection, with the best model yielding the predicted value of the parameter $X_i^{pred}$ solely based on the predictors enlisted in the step (e);

(g) a set of pre-corrected pupillometry parameters is calculated based on the formula

$$X_i^{pre-corr} = X_i^{mes} - X_i^{pred} + X_i^{avg}$$

, where $X_i^{avg}$ is a cohort average value calculated in the step (d), $X_i^{pred}$ is a value predicted by the best model selected in the step (f), while $X_i^{mes}$ is a parameter value extracted directly from the pupillary light reaction measurement;

(h) a set of light corrected pupillometry parameters

$$X_i^{corr} = \alpha X_i^{pre-corr} + (1-\alpha)X_i^{mes}$$

is calculated, where $\alpha \in$ [0,1], $X_i^{pred}$ is a value predicted by the best model selected in the step (f), while $X_i^{mes}$ being a parameter value extracted directly from the pupillary light reaction measurement;

(i) a pupil reactivity numerical scale invulnerable to external light conditions is constructed as a linear combination of numerical constant, and the product of light corrected pupillometry parameters i.e.

$$RI = C + \sum_i A_i X_i^{corr}$$

,

where $X_1^{corr} = X_1^{mes}$ denotes measured INIT, $X_2^{corr}$ denotes light corrected END, $X_3^{corr}$ denotes light corrected CAMP, $X_4^{corr}$ denotes light corrected ACV, $X_5^{corr}$ denotes light corrected ADV, $X_6^{corr}$ denotes light corrected MCV, $X_7^{corr}$ denotes light corrected PDV, $X_8^{corr}$ denotes light corrected DELTA, $X_9^{corr}$ denotes light corrected FIN, $X_{10}^{corr}$ denotes light corrected LAT, $X_{11}^{corr}$ denotes light corrected FT75, $X_{12}^{corr}$ denotes light corrected DAMP, whereas $\boldsymbol{C}$ and $\boldsymbol{A_i}$ are real-valued constants;

(j) to find a set of coefficients **C** and **A$_i$** a D2 dataset is used with a particular set of coefficient values found using a logistic regression fitted to the data with elastic-net regularization and additional light invariance regularisation;

(k) a light invariant reactivity index **NPx** is calculated by feeding a sigmoid function with **RI** such that the value of **NPx**, is within a predefined numerical range, preferably in the range from 0 to 5.

Preferably, in the steps (b) and , (d-i) at least one static pupillometric parameters selected from parameters: INIT, END, CAMP, DELTA, FIN, LAT, FT75, DAMP are only considered, and none of the dynamic parameters are used.

Preferably, in the steps (b) and (d-i) at least one dynamic pupillometric parameters selected from parameters: PDV, ACV, MCV, ADV are only considered, and none of the static parameters are used.

Preferably, in a step (e) image intensity in the video recording are taken into account as predictors instead of ISO and $\tau$.

Preferably, in step (i) $X^{mes}$ parameters are used instead of $X^{corr}$.

Preferably, the diagnostic device is a smartphone.

Preferably, datasets D1 and D2 are supplemented with additional data after each measurement result in order to retrain predictive models $X_i^{pred}$ mentioned in claim 1 steps (e) and (f) as well as improve a set of coefficients mentioned in claim 1 step (j).

Preferably, in a step (i) the parameter values are in the range $C \in [-50,50]$ and $A_i \in [-10,10],$

The second invention relates to a computer program comprising instructions which, when the program is executed on a computer, cause the computer to carry out the method according to the first invention.

The third invention relates to a computer-readable medium comprising the computer program according to the second invention.

The fourth invention relates to a system implementing the machine learning model according to any claims 1-8 for automatically diagnosing abnormal pupil behaviour based on assessing a pupil reactivity of a patient by assigning a numerical scale, said system comprising a memory, a processor, a camera, a light source, a light sensor and a digital display, wherein the system is configured and programmed for assessing pupil reactivity of the patient by the following steps:

(a) physical parameters of light sensor including the sensitivity ISO and/or exposure time $\tau$ is read out from a camera forming a single parameter ISOtau = ISO*$\tau$; (b) a pupillary light reaction measurement is performed using a diagnostic device on a single subject (patient)

(c) based on the measured pupillogram, at least one parameter selected from a set of measured pupillometry parameters $X^{mes}$ including $X_1^{mes} = \text{INIT}, X_2^{mes} = \text{END}, X_3^{mes} = \text{CAMP}$,

$X_4^{mes} = \text{ACV}, X_5^{mes} = \text{ADV}, X_6^{mes} = \text{MCV}, X_7^{mes} = \text{PDV}, X_8^{mes} = \text{DELTA}, X_9^{mes} = \text{FIN}, X_{10}^{mes} = \text{LAT}$

, $X_{11}^{mes} = \text{FT75}, X_{12}^{mes} = \text{DAMP}$ , is extracted from the measurement;

(d) a set of light corrected pupillometry parameters $X_i^{corr}$ is retrieved based on the real time physical measurements and measured pupillometry parameters $X_i^{mes}$ as well as a set of best predictive models mentioned in claim 1 (f);

(e) a pupil reactivity index invulnerable to external light conditions is calculated as a linear combination of numerical constant, and light corrected pupillometry parameters, i.e. $$RI = C + \sum_i A_i X_i^{corr}$$ , wherein $X_1^{corr} = X_1^{mes}$ denotes measured INIT, $X_2^{corr}$ denotes light corrected END, $X_3^{corr}$ denotes light corrected CAMP, $X_4^{corr}$ denotes light corrected ACV, $X_5^{corr}$ denotes light corrected ADV, $X_6^{corr}$ denotes light corrected MCV, $X_7^{corr}$ denotes light corrected PDV, $X_8^{corr}$ denotes light corrected DELTA and $X_9^{corr}$ denotes light corrected FIN, $X_{10}^{corr}$ denotes light corrected LAT, $X_{11}^{corr}$ denotes light corrected FT75, $X_{12}^{corr}$ denotes light corrected DAMP, whereas **C** and **$A_i$** are real-valued constants resulting from the machine learning model training;

(f) a light invariant reactivity index **NPx** is calculated by feeding a sigmoid function with **RI** such that the value of **NPx** is within a predefined numerical range, preferably in the range from 0 to 5, and optionally displaying the value of NPx on the display;

(g) based on the **NPx** threshold the measured pupil is classified as reactive or non-reactive and the device informs the operator on the non-reactivity of the pupil by activating sound and/or visual alarm on the diagnostic device.

Preferably, in step (e) $X_i^{mes}$ parameters are used instead of $X_i^{corr}$ .

Preferably, in the parameter values are in the range **C $\in$ [-50,50]** and $A_i \in$ **[-10,10],**

The fifth invention relates to a computer program comprising instructions which, when the program is executed in the system according to any one of the preceding claims, enable the system to automatically diagnose condition of a patient in a numerical scale from 0 to 5 by carrying out the steps specified in the fourth invention.

The sixth invention relates to a computer-readable medium comprising the computer program according to the fifth invention.

The seventh invention relates to a method for generating the Neuro-Pupillary index (NPx) using the dataset D2 acquired during pharmacological intervention, comprising:

◦ creating a plurality of of light-invariant pupillometry parameters;
◦ acquiring the dataset D1 under controlled light conditions, involving:

1. Measuring ambient light intensity using a calibrated sensor;
2. Recording pupillary light reflex (PLR) responses in subjects under varying light conditions;
3. Altering light conditions systematically to span a predetermined range of intensities;
4. Repeating measurements across a diverse cohort of subjects to ensure robust data representation;
5. Developing a predictive model that estimates PLR parameters, utilizing functions of measured light intensity as predictors;

◦ conducting ambient light measurements and PLR assessments in a similar manner as described in claim 1;
◦ administering a pharmacologically active agent known to influence PLR to subjects;
◦ repeating the measurement process under varying light conditions and across different subjects post-administration to capture drug-induced PLR variations;
◦ creating a predictive model that accounts for the drug's effect on PLR, using functions of light intensity and light-corrected PLR parameters as predictors.

The eight invention relates to a method for measuring the NPx by measuring ambient light conditions, PLR responses, and utilizing light-corrected parameters to calculate the index, followed by its display on a mobile device.

**Advantageous effects of the invention**

The invention according to the present patent specification has numerous advantages over the existing solutions. The use of pupil reactivity assessment according to the machine learning approach implemented in the invention is likely to be more useful than the state-of-the-art thanks to its significantly reduced external light variability. This allows for a more reliable separation between two patient groups based on their pupil reactivy measured within uncontrolled external light conditions. Moreover, a machine learning approach allows for constantly improving the precision of reactivity assessment and group separation by expanding the traning datasets (D1 and D2). Widespread adoption of disclosed light-corrected pupil reactivity index is an important step towards the standarization of PLR measurements performed accross clinics and research groups.

Advantageously, the present patent documentation discloses a full mathematical apparatus used for a practical application of Neuro-Pupillary index (NPx) on a mobile device such as smartphone. This machine-learning approach presents fully reliable tool that is useful for medics to quickly assess patient condition using numerical range. The invention can be simply implemented on any mobile device (including a smartphone), without need of buying any additional stand-alone medical hardware device (such as US9402542B2 or WO2010062400A1).

**Possible areas of application of the invention**

The present invention finds a primary use in a broad field of pupillometry, ophthalmology and neurological studies. A pupillary light response is nowadays studied within the context of other technological fields, such as medicine, automotive, virtual reality, gaming, education, drug development, quick drug detection in law enforcement etc. The visible light pupillometry is prone to the variability resulting from non-controlled light measurements conditions. The construction of light pupil reactivity invulnerable to external light might play a crucial role in increasing a sensitivity of prospective algorithms designed for neurodegenerative disorders diagnostics and tracking their progress. It might also find a use in ICU and critical care environment where the pupil reactivity assessment is a crucial part of the diagnostics and triage procedures.

**Preferred embodiments of the invention**

Now, the invention will be presented in more detail, in a preferred embodiment, with reference to the attached drawing in which:

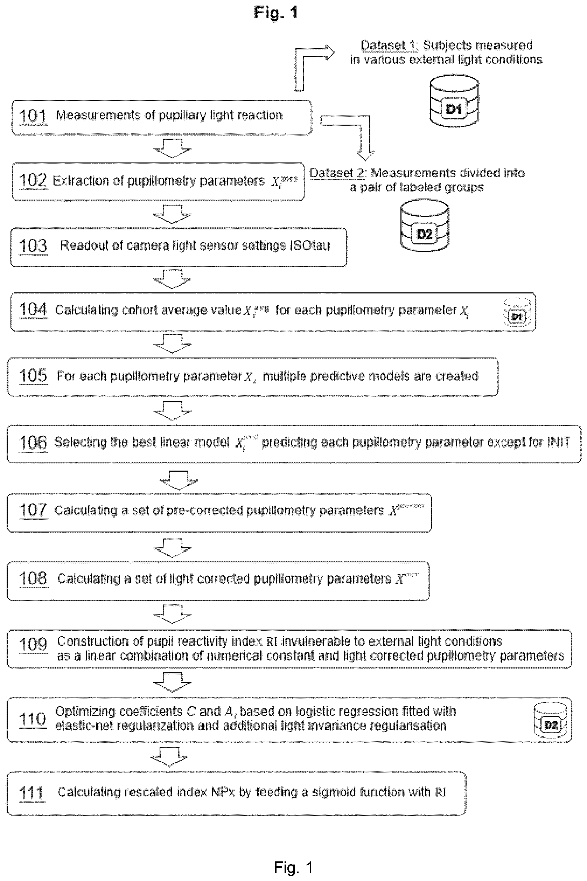

**Fig. 1** presents a general overview of method steps leading to the training of machine learning model for pupil reactivity assessment invulnerable to external light conditions.

**Fig. 2** (a) presents the results of average pupillary light reaction measurements performed on the cohort of 9 healthy subjects (dataset D1) with external light conditions spanning the range of 5-8600 lux. Uncertainty of the measurement is represented by a shaded area. (b) presents the results of average pupillary light reaction measurements performed on another cohort of 15 healthy subjects, with each subject subjected to the administration of tropicamide and the reactivity measurements are performed before and after the drug administration (dataset D2)

**Fig. 3** presents extracted canonical pupillometry parameters INIT, END, FIN, MCV, PDV, CAMP from the measurements performed on the cohort of 9 healthy subjects with external light conditions spanning the range of 5-10000 lux

**Fig. 4** presents the readout of light sensor parameters including the sensitivity ISO and exposure time $\tau$ for each of the measured subject and external light conditions spanning the range 5-10000 lux

**Fig. 5** presents selected light corrected pupillometry parameters aggregated over the measured subjects compared with the uncorrected pupillometric parameters

**Fig. 6** presents the values of pupil reactivity index invulnerable to external light conditions calculated on the cohort of 9 healthy subjects with external light conditions spanning the range of 5-10000 lux. In panel (a) the machine learning model leading to reactivity index has been trained according to embodiment 1, in panel (b) the machine learning model leading to reactivity index has been trained according to embodiment 2, in panel (c) the machine learning model leading to reactivity index has been trained according to embodiment 3

**Fig. 7** (a) presents a system implementing a method for automatically diagnosing abnormal pupil behaviour through assigning a numerical scale to pupil reactivity (b) presents a general idea of utilizing measured pupillogram for calculating external light invariant reactivity index by utilizing trained machine learning model.

**Fig. 8** presents an application of reactivity index for the observation of reactivity loss, where each subject has been measured before (pre) and after (post) drug administration. Used reactivity index is based on the machine learning model trained according to embodiement 1. A threshold of NPx = 3 allow for unambigous classification of a measurement belonging either to the first or the second group.

**Fig. 9** presents a sigmoid function mapping a pupil reactivity index invulnerable to external light conditions RI to the rescaled version of the index NPx that spans the numerical range 0-5

**Embodiment 1**

**A method for training a machine learning model for automatically diagnosing abnormal pupil behaviour through assigning a numerical scale to pupil reactivity, relying on the combination of dynamic and static pupillometric parameters and assessment of external light intensity**

In the following example a training of machine learning model for assessing pupil reactivity invulnerable to external light conditions is realised by performing a series of pupillary light reaction measurements for various external light conditions spanning 5-10000 lux on the cohort of 9 subjects. These measurements are forming the dataset D1.

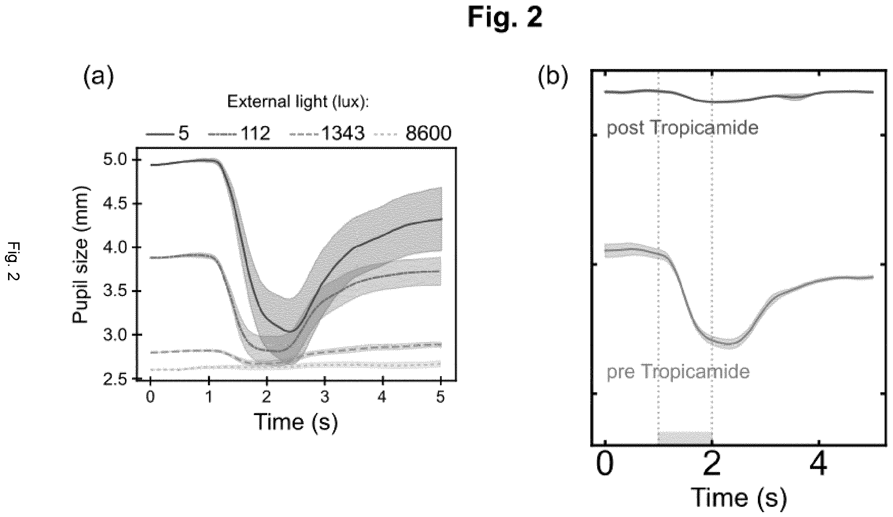

The averaged pupillary light reactions measured under various external light conditions are presented in the Figure 2 (a). The influence of external light on the observed pupillogram is clearly visible, for instance the amplitude of pupil contraction is heavily reduced for increased external light intensity. A second series of pupillary light reaction measurements is performed on another cohort of 15 subjects with each subject undergoing the administration of tropicamide. The reactivity measurements are performed before and after the drug administration forming the dataset D2 which is presented in the Figure 2 (b).

Afterwards for each measurement a set of pupillometry parameters is extracted comprising

$$X_1^{mes} = \text{INIT}, \quad X_2^{mes} = \text{END}, \quad X_3^{mes} = \text{CAMP}, \quad X_6^{mes} = \text{MCV}, \quad X_7^{mes} = \text{PDV}, \quad X_9^{mes} = \text{FIN}$$

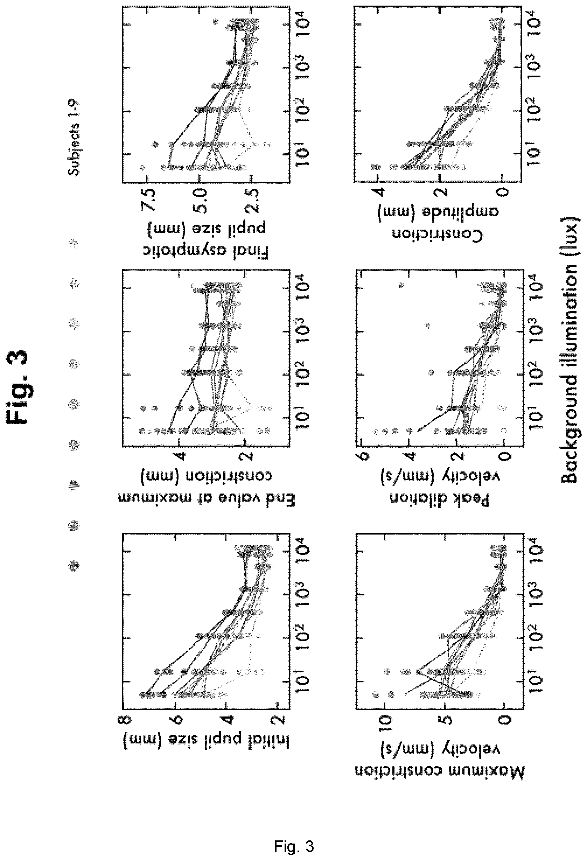

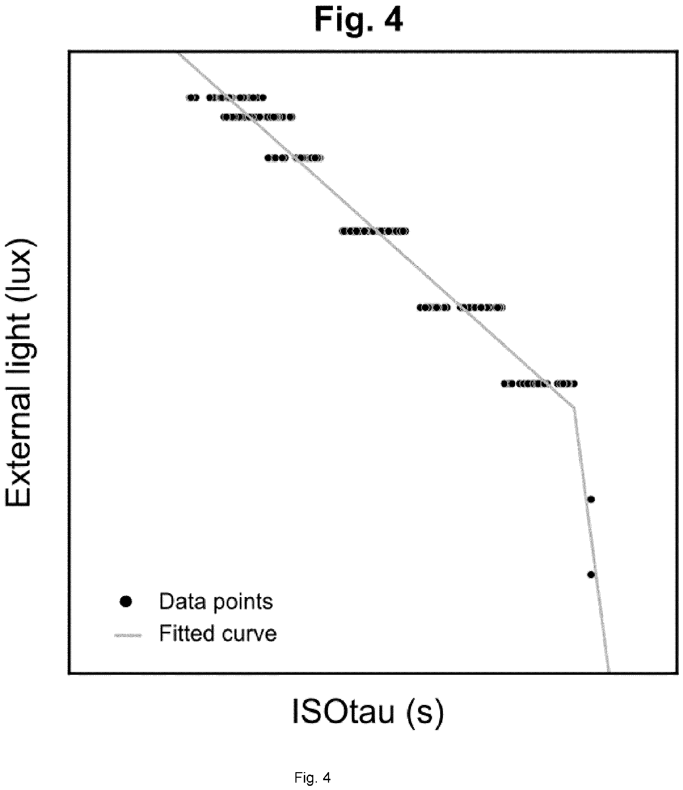

. Some of extracted pupillometric parameters are presented in the Figure 3. The dependence of pupillometric parameters on the external light intensity is especially pronounced for parameters INIT, CAMP and MCV - all of them significantly decreasing under increasing external light intensity. Similarly for each measurement light sensor parameter ISOtau is read out from the camera settings. The dependence of ISOtau on the external light is presented in Figure 4, with a clear anti-correlation visible between ISOtau and external light level.

In the next step a cohort average $X_i^{avg}$ is calculated based on the dataset D1 for each pupillometry parameter $X_i$ except for ($X_1$ = INIT) yielding a set of values summarized in the table presented below:

| Parameter | Variable name | Cohort average value |
|-----------|---------------|----------------------|
| INIT | $X_1$ | **Not applicable** |
| END | $X_2$ | $X_2^{avg}$ = 2.771 mm |
| CAMP | $X_3$ | $X_3^{avg}$ = 0.844 mm |
| MCV | $X_6$ | $X_6^{avg}$ = 1.986 mm/s |
| PDV | $X_7$ | $X_7^{avg}$ = 0.839 mm/s |
| FIN | $X_9$ | $X_9^{avg}$ = 3.403 mm |

Table 1: Calculated cohort average $X_i^{avg}$ based on the dataset D1 for each pupillometric parameter $X_i$ except for $X_1$ = INIT

In the discussed embodiement for each canonical pupillometry parameter $X_i$ (except for $X_1$ = INIT) a multiple predictive models are created were each model is a linear combination of the predictors ISOtau, INIT, their logarithms, squares, cubes, reciprocals as well as the pairwise products of before-mentioned. The selection of the best model for each pupillometry parameter $X_i$ (predicting its behaviour in a most reliable manner) is realised by selecting the model with the lowest Bayesian information criterion (BIC). The selection yields a following list of best predictive models each model responsible for predicting a behaviour of a single pupillometric parameter:

**Table 2: Best model for each selected pupillometric parameter $X_i$**

| Parameter | Variable name | Best predictive model |
|---|---|---|
| INIT | $X_1$ | Not applicable (INIT is one of a predictors) $X_1^{\text{pred}} = X_1^{\text{mes}}$ |
| END | $X_2$ | $X_2^{\text{pred}} =$ 0.97 + 0.59*INIT - 0.00062*INIT$^2$ - 0.00018*ISOtau$^2$ - 0.078*Log(INIT)*Log(ISOtau) |
| CAMP | $X_3$ | $X_3^{\text{pred}} =$ -0.42 + 0.13*INIT + 0.029*INIT$^2$ + 0.00016*ISOtau$^2$ + 0.098*Log(INIT)*Log(ISOtau) |
| MCV | $X_6$ | $X_6^{\text{pred}} =$ -1.5 + 1.0047*INIT - 0.93*Log(ISOtau) - 0.103*INIT$^2$ + 1.193*Log(INIT)*Log(ISOtau) |
| PDV | $X_7$ | $X_7^{\text{pred}} =$ -0.00020*ISOtau$^2$ + 0.16*ISOtau$^{-1}$ + 0.36*Log(INIT)*Log(ISOtau) |
| FIN | $X_9$ | $X_9^{\text{pred}} =$ 0.46 + 1.97*Log(INIT) + 0.041*INIT$^2$-0.00014*ISOtau$^2$ .46 + 1.97*Log(INIT) + |

After the selection of the best model for each pupillometric parameter $X_i$ (except for a predictor parameter $X_1$ = INIT) a set of light pre-corrected parameter values is calculated using a following formula:

$$X_i^{\text{pre-corr}} = X_i^{\text{mes}} - X_i^{\text{pred}} + X_i^{\text{avg}}$$

It is worth mentioning that for the individuals which reaction perfectly follows the predictive model, the value of pre-corrected parameter is equal to the population average.

Afterwards a set of light corrected parameter values is calculated using a formula:

$$X_i^{\text{corr}} = \alpha X_i^{\text{pre-corr}} + (1 - \alpha) X_i^{\text{mes}}$$

with the factor $\alpha$ = 1 yielding:

$$X_i^{\text{corr}} = X_i^{\text{pre-corr}}$$

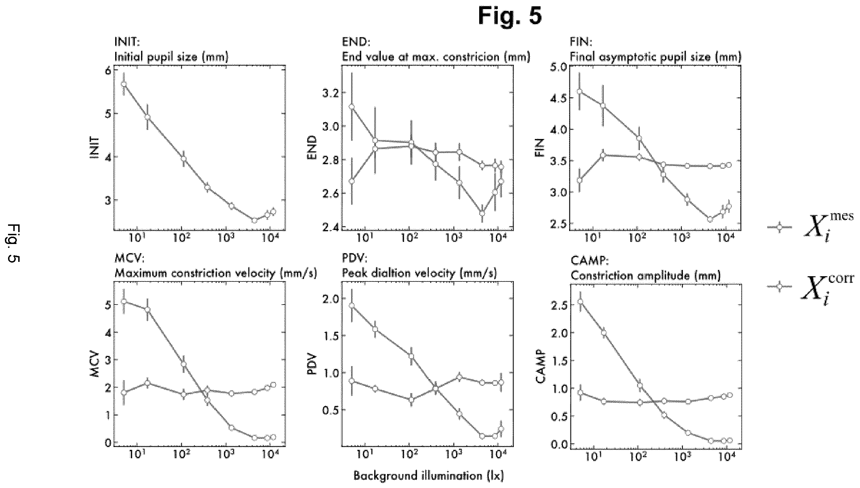

In general this step might involve another form of factor $\alpha \in$ **[0,1]**. Selected light corrected parameters are presented in Fig. 5.

Finally pupil reactivity index invulnerable to external light conditions is constructed as a following linear combination of light corrected parameters (except for a predictor parameter $X_1^{\text{corr}} = X_1^{\text{mes}} =$ INIT):

$$\text{RI} = C + \sum_i A_i X_i^{\text{corr}}$$

The exact values of coefficients **C** and $A_i$ are found using a logistic regression with elastic-net regularization fitted to the data from the dataset D2 and additional light invariance regularisation. The procedure yields a following set of non-zero coefficients:

**Table 3: Numerical value of non-zero coefficients used for calculating reactivity index RI relying on the combination of dynamic and static pupillometric parameters**

| Coefficient | Numerical Value |
|---|---|
| $C$ | $8.64 \times 10^{-11}$ |
| $A_1$ | -0.0066 |
| $A_2$ | 2.44 |
| $A_3$ | -2.60 |
| $A_6$ | -0.84 |
| $A_7$ | -1.24 |
| $A_9$ | -2.77 |

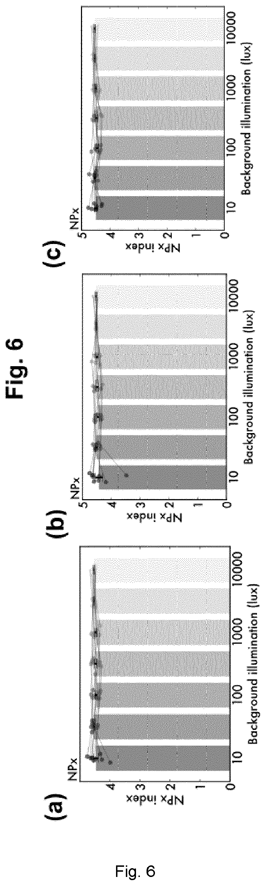

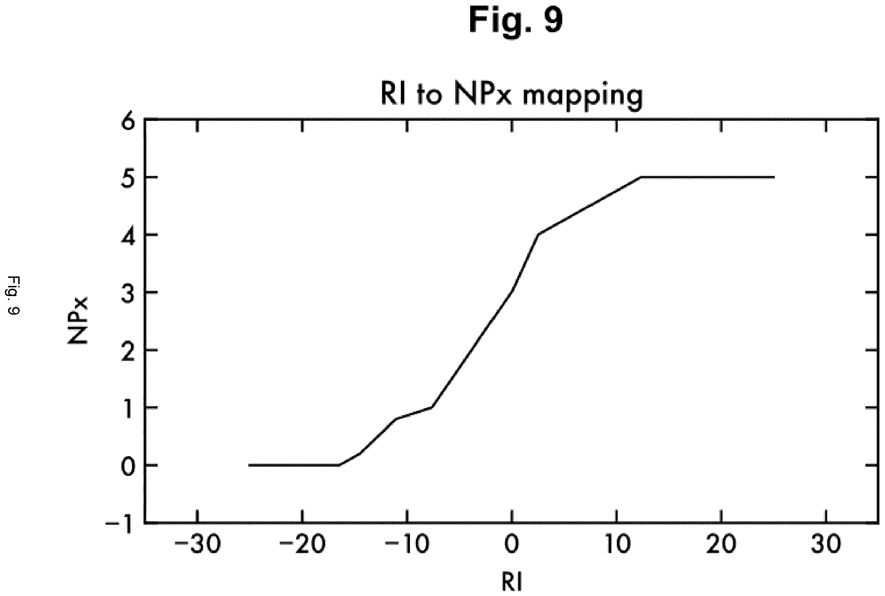

Once pupil reactivity index **RI** is found, one might calculate the rescaled version of the index that spans the numerical range 0-5 by feeding a sigmoid function with reactivity index **RI** . In this embodiment a piecewise linear sigmoid function has been used of the form presented in Figure 9. This index shall be refered as **NPx** (Neuro-Pupillary Index). In Figure 6 (a) we present the values of **NPx** calculated under various external light conditions for the measurements of the cohort of 9 individuals forming a dataset D1. It is clearly visible that the assessment of pupil reactivity remains stable across the entire range of external light.

**Embodiment 2**

**A method for training a machine learning model for automatically diagnosing abnormal pupil behaviour through assigning** a **numerical scale to pupil reactivity, relying on the combination of static pupillometric parameters and assessment of external light intensity**

In the following example a training of machine learning model for assessing pupil reactivity invulnerable to external light conditions is realised by performing a series of pupillary light reaction measurements for various external light conditions spanning 5-10000 lux on the cohort of 9 subjects. These measurements are forming the dataset D1.

The averaged pupillary light reactions measured under various external light conditions are presented in the Figure 2 (a). The influence of external light on the observed pupillogram is clearly visible, for instance the amplitude of pupil contraction is heavily reduced for increased external light intensity. A second series of pupillary light reaction measurements is performed on another cohort of 15 subjects with each subject undergoing the administration of tropicamide. The reactivity measurements are performed before and after the drug administration forming the dataset D2 which is presented in the Figure 2 (b).

Afterwards for each measurement a set of pupillometry parameters is extracted comprising

$$X_1^{mes} = \text{INIT}, \quad X_2^{mes} = \text{END}, \quad X_3^{mes} = \text{CAMP}, \quad X_8^{mes} = \text{DELTA}, \quad X_9^{mes} = \text{FIN}, \quad X_{12}^{mes} = \text{DAMP}$$ . Some

of extracted pupillometric parameters are presented in the Figure 3. The dependence of pupillometric parameters on the external light intensity is especially pronounced for parameters INIT, CAMP - all of them significantly decreasing under increasing external light intensity. Similarly for each measurement light sensor parameter ISOtau is read out from the camera settings. The dependence of ISOtau on the external light is presented in Figure 4, with a clear anti-correlation visible between ISOtau and external light level.

In the next step a cohort average $X_i^{avg}$ is calculated based on the dataset D1 for each pupillometry parameter $X_i$ except for ($X_1$ = INIT) yielding a set of values summarized in the table presented below:

| Parameter | Variable name | Cohort average value |
|---|---|---|
| INIT | $X_1$ | Not applicable |
| END | $X_2$ | $X_2^{avg}$ = 2.771 mm |

(continued)

| Parameter | Variable name | Cohort average value |
|---|---|---|
| CAMP | $X_3$ | $X_3^{avg}$ = 0.844 mm |
| DELTA | $X_8$ | $X_8^{avg}$ = 18.375 |
| FIN | $X_9$ | $X_9^{avg}$ = 3.403 mm |
| DAMP | $X_{12\backslash}$ | $X_{12}^{avg}$ = 0.632 mm |

Table 4: Calculated cohort average $X_i^{avg}$ based on the dataset D1 for each pupillometric parameter $X_i$ except for $X_1$ = INIT

In the discussed embodiement for each canonical pupillometry parameter $X_i$ (except for $X_1$ = INIT) a multiple predictive models are created were each model is a linear combination of the predictors ISOtau, INIT, their logarithms, squares, cubes, reciprocals as well as the pairwise products of before-mentioned. The selection of the best model for each pupillometry parameter $X_i$ (predicting its behaviour in a most reliable manner) is realised by selecting the model with the lowest Bayesian information criterion (BIC). The selection yields a following list of best predictive models each model responsible for predicting a behaviour of a single pupillometric parameter:

Table 5: Best model for each selected pupillometric parameter $X_i$

| Parameter | Variable name | Best predictive model |
|---|---|---|
| INIT | $X_1$ | **Not applicable (INIT is one of a predictors)** $X_1^{pred} = X_1^{mes}$ |
| END | $X_2$ | $X_2^{pred} =$ 0.97 + 0.59*INIT - 0.00062*INIT$^2$ - 0.00018*ISOtau$^2$ - 0.078*Log(INIT)*Log(ISOtau) |
| CAMP | $X_3$ | $X_3^{pred} =$ -0.42 + 0.13*INIT + 0.029*INIT$^2$ + 0.00016*ISOtau$^2$ + 0.098*Log(INIT)*Log(ISOtau) |
| DELTA | $X_8$ | $X_8^{pred} =$ -6.64 + 0.011*ISOtau$^2$ + 6.56*Log(INIT)$^2$ - 0.34*ISOtau$^{-2}$ + 4.52*ISOtau$^{-1}$-0.17*INIT*ISOtau + 7.07*Log(INIT)*Log(ISOtau) |
| FIN | $X_9$ | $X_9^{pred} =$ 0.46+1.97*Log(INIT)+0.41*INIT$^2$ -0.00013*ISOtau$^2$ |
| DAMP | $X_{12}$ | $X_{12}^{pred} =$ 0.25*Log(INIT)$^2$+0.041*ISOtau$^{-1}$ - 0.15*Log(INIT)$^2$ +0.13*Log(INIT)*Log(ISOtau) |

After the selection of the best model for each pupillometric parameter $X_i$ (except for a predictor parameter $X_1$ = INIT) a set of light pre-corrected parameter values is calculated using a following formula:

$$X_i^{pre-corr} = X_i^{mes} - X_i^{pred} + X_i^{avg}$$

It is worth mentioning that for the individuals which reaction perfectly follows the predictive model, the value of pre-corrected parameter is equal to the population average.

Afterwards a set of light corrected parameter values is calculated using a formula:

$$X_i^{\text{corr}} = \alpha X_i^{\text{pre-corr}} + (1 - \alpha) X_i^{\text{mes}}$$

with the factor $\alpha = 1$ yielding:

$$X_i^{\text{corr}} = X_i^{\text{pre-corr}}$$

In general this step might involve another form of factor $\alpha \in$ **[0,1]**. Selected light corrected parameters are presented in Fig. 5.

Finally pupil reactivity index invulnerable to external light conditions is constructed as a following linear combination of light corrected parameters (except for a predictor parameter $X_1^{\text{corr}} = X_1^{\text{mes}} =$ INIT):

$$\text{RI} = C + \sum_i A_i X_i^{\text{corr}}$$

The exact values of coefficients $C$ and $A_i$ are found using a logistic regression with elastic-net regularization fitted to the data from the dataset D2 and additional light invariance regularisation. The procedure yields a following set of non-zero coefficients:

**Table** 6: **Numerical value of non-zero coefficients used for calculating reactivity index RI relying on the combination of static pupillometric parameters**

| Coefficient | Numerical Value |
|---|---|
| $C$ | $3.18 \times 10^{-7}$ |
| $A_1$ | -0.0304 |
| $A_2$ | 0.03 |
| $A_3$ | -0.84 |
| $A_8$ | -0.14 |
| $A_9$ | -0.35 |
| $A_{12}$ | -5.26 |

Once pupil reactivity index **RI** is found, one might calculate the rescaled version of the index that spans the numerical range 0-5 by feeding a sigmoid function with reactivity index **RI**. In this embodiment a piecewise linear sigmoid function has been used of the form presented in Figure 9. This index shall be refered as **NPx** (Neuro-Pupillary Index). In Figure 6 (b) we present the values of **NPx** calculated under various external light conditions for the measurements of the cohort of 9 individuals forming a dataset D1. It is clearly visible that the assessment of pupil reactivity remains stable across the entire range of external light.

**Embodiment 3**

**A method for training a machine learning model for automatically diagnosing abnormal pupil behaviour through assigning a numerical scale to pupil reactivity, relying on the combination of dynamic pupillometric parameters and assessment of external light intensity**

In the following example a training of machine learning model for assessing pupil reactivity invulnerable to external light conditions is realised by performing a series of pupillary light reaction measurements for various external light conditions spanning 5-10000 lux on the cohort of 9 subjects. These measurements are forming the dataset D1.

The averaged pupillary light reactions measured under various external light conditions are presented in the Figure 2 (a). The influence of external light on the observed pupillogram is clearly visible, for instance the amplitude of pupil contraction is heavily reduced for increased external light intensity. A second series of pupillary light reaction measurements is performed on another cohort of 15 subjects with each subject undergoing the administration of tropicamide. The reactivity measurements are performed before and after the drug administration forming the dataset D2 which is presented in the Figure 2 (b).

Afterwards for each measurement a set of pupillometry parameters is extracted comprising $X_6^{mes}$ = MCV, $X_7^{mes}$ = PDV. Some of extracted pupillometric parameters are presented in the Figure 3. The dependence of pupillometric parameters on the external light intensity is especially pronounced for parameters MCV, PDV - all of them significantly decreasing under increasing external light intensity. Similarly for each measurement light sensor parameter ISOtau is read out from the camera settings. The dependence of ISOtau on the external light is presented in Figure 4, with a clear anti-correlation visible between ISOtau and external light level.

In the next step a cohort average $X_i^{avg}$ is calculated based on the dataset D1 for each pupillometry parameter $X_i$ except for ($X_1$ = INIT) yielding a set of values summarized in the table presented below:

| Parameter | Variable name | Cohort average value |
|---|---|---|
| INIT | $X_1$ | **Not applicable** |
| MCV | $X_6$ | $X_6^{avg}$ = 1.986 mm/s |
| PDV | $X_7$ | $X_7^{avg}$ = 0.839 mm/s |

Table 7: Calculated cohort average $X_i^{avg}$ based on the dataset D1 for each pupillometric parameter $X_i$ except for $X_1$ = INIT

In the discussed embodiement for each canonical pupillometry parameter $X_i$ (except for $X_1$ = INIT) a multiple predictive models are created were each model is a linear combination of the predictors ISOtau, INIT, their logarithms, squares, cubes, reciprocals as well as the pairwise products of before-mentioned. The selection of the best model for each pupillometry parameter $X_i$ (predicting its behaviour in a most reliable manner) is realised by selecting the model with the lowest Bayesian information criterion (BIC). The selection yields a following list of best predictive models each model responsible for predicting a behaviour of a single pupillometric parameter:

**Table 8: Best model for each selected pupillometric parameter $X_i$**

| Parameter | Variable name | Best predictive model |
|---|---|---|
| INIT | $X_1$ | **Not applicable (INIT is one of a predictors)** $X_1^{pred} = X_1^{mes}$ |
| MCV | $X_6$ | $X_6^{pred}$ = -1.5 + 1.0047*INIT - 0.93*Log(ISOtau) - 0.103*INIT$^2$ + 1.193*Log(INIT)*Log(ISOtau) |
| PDV | $X_7$ | $X_7^{pred}$ = -0.00020*ISOtau$^2$ + 0.16*ISOtau$^{-1}$ + 0.36*Log(INIT)*Log(ISOtau) |

After the selection of the best model for each pupillometric parameter $X_i$ (except for a predictor parameter $X_1$ = INIT) a set of light pre-corrected parameter values is calculated using a following formula:

$$X_i^{\text{pre-corr}} = X_i^{\text{mes}} - X_i^{\text{pred}} + X_i^{\text{avg}}$$

It is worth mentioning that for the individuals which reaction perfectly follows the predictive model, the value of pre-corrected parameter is equal to the population average.

Afterwards a set of light corrected parameter values is calculated using a formula:

$$X_i^{\text{corr}} = \alpha X_i^{\text{pre-corr}} + (1 - \alpha)X_i^{\text{mes}}$$

with the factor $\alpha$ **= 1** yielding:

$$X_i^{\text{corr}} = X_i^{\text{pre-corr}}$$

In general this step might involve another form of factor $\alpha \in$ **[0,1]**. Selected light corrected parameters are presented in Fig. 5.

Finally pupil reactivity index invulnerable to external light conditions is constructed as a following linear combination of light corrected parameters (except for a predictor parameter $X_1^{\text{corr}} = X_1^{\text{mes}} =$ INIT):

$$\text{RI} = C + \sum_i A_i X_i^{\text{corr}}$$

The exact values of coefficients **C** and **$A_i$** are found using a logistic regression with elastic-net regularization fitted to the data from the dataset D2 and additional light invariance regularisation. The procedure yields a following set of non-zero coefficients:

**Table** 9: **Numerical value of non-zero coefficients used for calculating** reactivity **index RI relying on the combination of dynamic pupillometric parameters**

| Coefficient | Numerical Value |
|---|---|
| **C** | -1.83 |
| **$A_6$** | -2.33 |
| **$A_7$** | -2.82 |

Once pupil reactivity index **RI** is found, one might calculate the rescaled version of the index that spans the numerical range 0-5 by feeding a sigmoid function with reactivity index **RI**. In this embodiment a piecewise linear sigmoid function has been used of the form presented in Figure 9. This index shall be refered as **NPx** (Neuro-Pupillary Index). In Figure 6 (c) we present the values of **NPx** calculated under various external light conditions for the measurements of the cohort of 9 individuals forming a dataset D1. It is clearly visible that the assessment of pupil reactivity remains stable across the entire range of external light.

**Embodiment 4**

**A system implementing a method for automatically diagnosing abnormal pupil behaviour through assigning a numerical scale to pupil reactivity invulnerable to external light conditions based on the trained machine learning model**

In the system implementing a method for automatically diagnosing abnormal pupil behaviour the assessment of pupil reactivity is realised by performing a pupillary light reaction measurement using a diagnostic device on a single subject. Additionally physical parameters of light sensor including the sensitivity ISO and/or exposure time $\tau$ is read out from a camera forming a single parameter ISOtau = ISO*$\tau$;

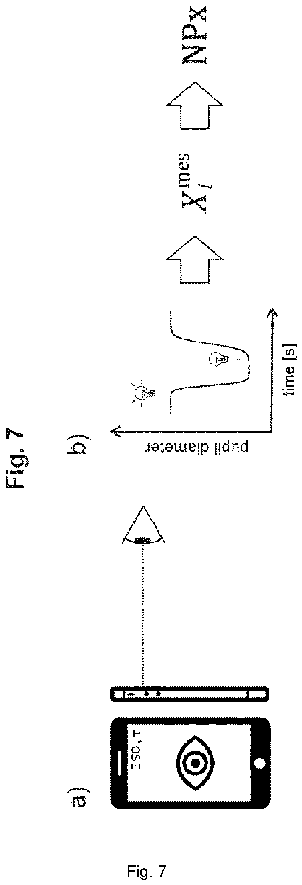

The system has been illustrated in the Figure 7(a). Afterwards a set of pupillometric parameters is extracted comprising $X_1^{\text{mes}}$ = INIT, $X_2^{\text{mes}}$ = END, $X_3^{\text{mes}}$ = CAMP, $X_6^{\text{mes}}$ = MCV, $X_7^{\text{mes}}$ = PDV $X_9^{\text{mes}}$ = FIN,

and a parameter of light sensor ISOtau .

A set of light pre-corrected parameter values is calculated using a following formula:

$$X_i^{\mathrm{pre-corr}} = X_i^{\mathrm{mes}} - X_i^{\mathrm{pred}} + X_i^{\mathrm{avg}}$$,

where cohort average values $X_i^{\mathrm{avg}}$ are presented in Table 1, while best predictive models for calculating $X_i^{\mathrm{pred}}$ are presented in Table 2. Similarly a set of light corrected parameter values is calculated using a formula below:

$$X_i^{\mathrm{corr}} = \alpha X_i^{\mathrm{pre-corr}} + (1 - \alpha) X_i^{\mathrm{mes}}$$

with the factor $\alpha = 1$ resulting in:

$$X_i^{\mathrm{corr}} = X_i^{\mathrm{pre-corr}}$$

Afterwards a pupil reactivity index invulnerable to external light conditions is calculated as a following linear combination of light corrected parameters (except for a predictor parameter $X_1^{\mathrm{corr}} = X_1^{\mathrm{mes}} = \mathrm{INIT}$ )

$$\mathrm{RI} = C + \sum_i A_i X_i^{\mathrm{corr}}$$ ,

where numerical values of coefficients **C** and $A_i$ have been obtained by traning a machine learning model according to the method presented in embodiment 1, and their non-zero values are presented in Table 3.

Once pupil reactivity index is found, one might calculate the rescaled version of the index that spans the numerical range 0-5 by feeding a sigmoid function with reactivity index **RI**. In this embodiment a piecewise linear sigmoid function has been used of the form presented in Figure 9. This index shall be refered as **NPx** (Neuro-Pupillary Index).

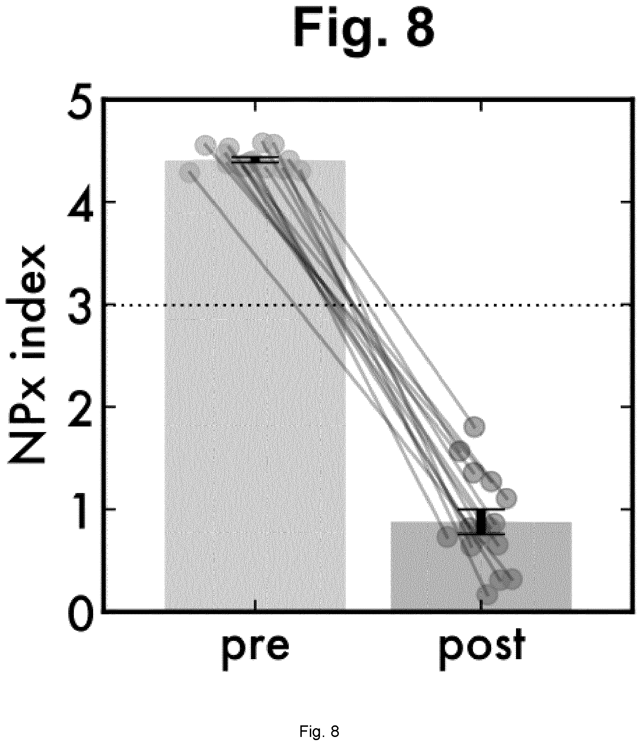

Based on the **NPx** threshold the measured pupil is classified as reactive or non-reactive. In Fig. 8 we present the assessement of pupil reactivity performed by a system revealing the pupil reactivity loss induced by the administration of tropcamide. In the illustrated scenario each subject has been measured before (pre) and after (post) the administration of the tropicamide modifying the reactivity of the pupil. It is clearly visible that the reactivity dropped down sharply following the expected influence of the drug. Each measurement can be unambigously classified to one of the measurment groups (pre or post) by reading out an **NPx** value and comparing it with a numerical thershold of **NPx = 3**. If the measured **NPx** value is below 3, the diagnostic device activates sound and visual alarm informing the user about pupil reaction abnormality.

****Additional information, related to the present invention and technical effects of the invention can be found in the application no.* EP 23461698.5 of 22 December 2023 - *the disclosure of which is hereby referred to and fully incorporated.*

**Claims**

1. **A method for training** a **machine learning model** for automatically diagnosing abnormal pupil behaviour through assigning a numerical scale to pupil reactivity, comprising the following steps:

   (a) a pupillary light reaction measurements are performed using a diagnostic device on the cohort of plurality of subjects under various external light conditions spanning the expected range of further external light invulnerability forming a first dataset (D1), similarly pupillary light reaction measurements are performed on the cohort of plurality of subjects with the measurements divided into a pair of labeled groups, with a first group of measurements performed before administration of drug reducing pupil reactivity (such as tropicamide or pilocarpine) and the second group of measurements performed after the drug administration, both forming a dataset (D2)

   (b) based on the measured pupillograms, at least one parameter selected from a set of measured pupillometry parameters $X^{\mathrm{mes}}$ including $X_1^{\mathrm{mes}} = \mathrm{INIT}$, $X_2^{\mathrm{mes}} = \mathrm{END}$, $X_3^{\mathrm{mes}} =$ CAMP,

$$X_4^{mes} = ACV, \quad X_5^{mes} = ADV, \quad X_6^{mes} = MCV, \quad X_7^{mes} = PDV, \quad X_8^{mes} = DELTA, \quad X_9^{mes} = FIN \quad,$$

$$X_{10}^{mes} = LAT, \quad X_{11}^{mes} = FT75, \quad X_{12}^{mes} = DAMP \quad, \text{ is extracted from each measurement;}$$

(c) parameters of light sensor reflecting external light intensity such as the sensitivity ISO and/or exposure time $\tau$ are read out from a camera forming a single parameter ISOtau = ISO*$\tau$;

(d) for each pupillometry parameter $X_i$ selected in step (b) (except for $X_1^{mes} = INIT$) its cohort average value is calculated based on the dataset D1, referred as $X_i^{avg}$ ;

(e) for each canonical pupillometry parameter $X_i$ (except for $X_1^{mes} = INIT$) a multiple predictive models are created were each model is a linear combination of the predictors ISOtau, INIT, their logarithms, squares, cubes, reciprocals as well as the pairwise products of before-mentioned giving in total $N$ predictors of a model, the predictive models are constructed using a binary feature inclusion with a total number of models equal $2^N$ ;

(f) the selection of the best model from all created models in step (e) predicting a pupillometry parameter $X_i$ relies on selecting the model with the lowest Bayesian information criterion (BIC) or similar method of model selection, with the best model yielding the predicted value of the parameter $X_i^{pred}$ solely based on the predictors enlisted in the step (e);

(g) a set of pre-corrected pupillometry parameters is calculated based on the formula

$$X_i^{pre-corr} = X_i^{mes} - X_i^{pred} + X_i^{avg} \quad, \text{ where } \quad X_i^{avg} \text{ is a cohort average value calculated in the step (d),}$$

$X_i^{pred}$ is a value predicted by the best model selected in the step (f), while $X_i^{mes}$ is a parameter value extracted directly from the pupillary light reaction measurement;

(h) a set of light corrected pupillometry parameters $X_i^{corr} = \alpha X_i^{pre-corr} + (1-\alpha) X_i^{mes}$ is calculated, where $\alpha \in [0,1]$, $X_i^{pred}$ is a value predicted by the best model selected in the step (f), while $X_i^{mes}$ being a parameter value extracted directly from the pupillary light reaction measurement;

(i) a pupil reactivity numerical scale invulnerable to external light conditions is constructed as a linear combination of numerical constant, and the product of light corrected pupillometry parameters i.e.

$$RI = C + \sum_i A_i X_i^{corr} \quad, \text{ where } \quad X_1^{corr} = X_1^{mes} \text{ denotes measured INIT, } X_2^{corr} \text{ denotes light corrected}$$

END, $X_3^{corr}$ denotes light corrected CAMP, $X_4^{corr}$ denotes light corrected ACV, $X_5^{corr}$ denotes light corrected ADV, $X_6^{corr}$ denotes light corrected MCV, $X_7^{corr}$ denotes light corrected PDV, $X_8^{corr}$ denotes light corrected DELTA, $X_9^{corr}$ denotes light corrected FIN, $X_{10}^{corr}$ denotes light corrected LAT, $X_{11}^{corr}$ denotes light corrected FT75, $X_{12}^{corr}$ denotes light corrected DAMP, whereas $C$ and $A_i$ are real-valued constants;

(j) to find a set of coefficients $C$ and $A_i$ a D2 dataset is used with a particular set of coefficient values found using a logistic regression fitted to the data with elastic-net regularization and additional light invariance regularisation;

(k) a light invariant reactivity index **NPx** is calculated by feeding a sigmoid function with **RI** such that the value of **NPx,** is within a predefined numerical range, preferably in the range from 0 to 5.

**2.** The method according to the claim 1, **characterized in that** in the steps (b) and , (d-i) at least one static pupillometric parameter selected from parameters: INIT, END, CAMP, DELTA, FIN, LAT, FT75, DAMP are only considered, and none of the dynamic parameters are used.

**3.** The method according to the claim 1, **characterized in that** in the steps (b) and (d-i) at least one dynamic pupillometric parameter selected from parameters: PDV, ACV, MCV, ADV are only considered, and none of the static parameters are used.

4. The method according to any of claims 1-3, **characterized in that** in a step (e) image intensity in the video recording are taken into account as predictors instead of ISO and $\tau$.

5. The method according to any of claims 1-4 **characterized in that** in step (i) $X^{mes}$ parameters are used instead of $X^{corr}$.

6. The method according to any one of the preceding claims, wherein the diagnostic device is a smartphone.

7. The method according to any one of the preceding claims, wherein datasets D1 and D2 are supplemented with additional data after each measurement result in order to retrain predictive models $X_i^{pred}$ mentioned in claim 1 steps (e) and (f) as well as improve a set of coefficients mentioned in claim 1 step (j).

8. The method according to claim 1, **characterized in that** in a step (i) the parameter values are in the range $C \in [-50,50]$ and $A_i \in$ **[-10,10], ,**

9. **A computer program comprising instructions which,** when the program is executed on a computer, cause the computer to carry out the method according to any one of claims 1 to 8.

10. **A computer-readable medium** comprising the computer program according to claim 9.

11. **A system implementing the machine learning model** according to any claims 1-8 for automatically diagnosing abnormal pupil behaviour based on assessing a pupil reactivity of a patient by assigning a numerical scale, said system comprising a memory, a processor, a camera, a light source, a light sensor and a digital display, wherein the system is configured and programmed for assessing pupil reactivity of the patient by the following steps:

(a) physical parameters of light sensor including the sensitivity ISO and/or exposure time $\tau$ is read out from a camera forming a single parameter ISOtau = ISO*$\tau$;
(b) a pupillary light reaction measurement is performed using a diagnostic device on a single subject (patient);
(c) based on the measured pupillogram, at least one parameter selected from a set of measured pupillometry parameters $X^{mes}$ including $X_1^{mes}$ = INIT, $X_2^{mes}$ = END, $X_3^{mes}$ = CAMP ,

$X_4^{mes}$ = ACV, $X_5^{mes}$ = ADV, $X_6^{mes}$ = MCV, $X_7^{mes}$ = PDV, $X_8^{mes}$ = DELTA, $X_9^{mes}$ = FIN, $X_{10}^{mes}$ = LAT , $X_{11}^{mes}$ = FT75, $X_{12}^{mes}$ = DAMP , is extracted from the measurement;

(d) a set of light corrected pupillometry parameters $X_i^{corr}$ is retrieved based on the real time physical measurements and measured pupillometry parameters $X_i^{mes}$ as well as a set of best predictive models;
(e) a pupil reactivity index invulnerable to external light conditions is calculated as a linear combination of numerical constant, and light corrected pupillometry parameters, i.e. $RI = C + \sum_i A_i X_i^{corr}$ , wherein $X_1^{corr} = X_1^{mes}$ denotes measured INIT, $X_2^{corr}$ denotes light corrected END, $X_3^{corr}$ denotes light corrected CAMP, $X_4^{corr}$ denotes light corrected ACV, $X_5^{corr}$ denotes light corrected ADV, $X_6^{corr}$ denotes light corrected MCV, $X_7^{corr}$ denotes light corrected PDV, $X_8^{corr}$ denotes light corrected DELTA and $X_9^{corr}$ denotes light corrected FIN, $X_{10}^{corr}$ denotes light corrected LAT, $X_{11}^{corr}$ denotes light corrected FT75, $X_{12}^{corr}$ denotes light corrected DAMP, whereas $C$ and $A_i$ are real-valued constants resulting from the machine learning model training;
(f) a light invariant reactivity index **NPx** is calculated by feeding a sigmoid function with **RI** such that the value of **NPx** is within a predefined numerical range, preferably in the range from 0 to 5, and optionally displaying the value of NPx on the display;

(g) based on the **NPx** threshold the measured pupil is classified as reactive or non-reactive and the device informs the operator on the non-reactivity of the pupil by activating sound and/or visual alarm on the diagnostic device.

12. The system according to any of claims 11 **characterized in that** in step (e) $X_i^{mes}$ parameters are used instead of $X_i^{corr}$.

13. The system according to claim 11 or 12, **characterized in that** the parameter values are in the range $C \in$ **[-50,50]** and $A_i \in$ **[-10,10],**

14. A computer program comprising instructions which, when the program is executed in the system according to any one of the preceding claims, enable the system to automatically diagnose condition of a patient in a numerical scale from 0 to 5 by carrying out the steps specified in any one of claims 11 to 13.

15. A computer-readable medium comprising the computer program according to claim 14.

16. A method for generating the Neuro-Pupillary index (NPx) using the dataset D2 acquired during pharmacological intervention, comprising:

   ○ creating a plurality of light-invariant pupillometry parameters;
   ○ acquiring the dataset D1 under controlled light conditions, involving:

   1. Measuring ambient light intensity using a calibrated sensor;
   2. Recording pupillary light reflex (PLR) responses in subjects under varying light conditions;
   3. Altering light conditions systematically to span a predetermined range of intensities;
   4. Repeating measurements across a diverse cohort of subjects to ensure robust data representation;
   5. Developing a predictive model that estimates PLR parameters, utilizing functions of measured light intensity as predictors;

   ○ conducting ambient light measurements and PLR assessments in a similar manner as described in claim 1;
   ○ administering a pharmacologically active agent known to influence PLR to subjects;
   ○ repeating the measurement process under varying light conditions and across different subjects post-administration to capture drug-induced PLR variations;
   ○ creating a predictive model that accounts for the drug's effect on PLR, using functions of light intensity and light-corrected PLR parameters as predictors.

17. A method for measuring the NPx by measuring ambient light conditions, PLR responses, and utilizing light-corrected parameters to calculate the index, followed by its display on a mobile device.

## Fig. 1

Dataset 1: Subjects measured
in various external light conditions

**D1**

101  Measurements of pupillary light reaction

102  Extraction of pupillometry parameters $X_i^{mes}$

Dataset 2: Measurements divided into
a pair of labeled groups

**D2**

103  Readout of camera light sensor settings ISOtau

104  Calculating cohort average value $X_i^{avg}$ for each pupillometry parameter $X_i$   **D1**

105  For each pupillometry parameter $X_i$ multiple predictive models are created

106  Selecting the best linear model $X_i^{pred}$ predicting each pupillometry parameter except for INIT

107  Calculating a set of pre-corrected pupillometry parameters $X^{pre-corr}$

108  Calculating a set of light corrected pupillometry parameters $X^{corr}$

109  Construction of pupil reactivity index RI invulnerable to external light conditions
as a linear combination of numerical constant and light corrected pupillometry parameters

110  Optimizing coefficients $C$ and $A_i$ based on logistic regression fitted with
elastic-net regularization and additional light invariance regularisation   **D2**

111  Calculating rescaled index NPx by feeding a sigmoid function with RI

Fig. 1

Fig. 2

Fig. 3

# Fig. 4

Fig. 4

Fig. 5

Fig. 6

Fig. 7

# Fig. 8

Fig. 8

Fig. 9

Europäisches
Patentamt

European
Patent Office

Office européen
des brevets

**EUROPEAN SEARCH REPORT**

Application Number

EP 23 46 1699

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/137614 A2 (NEUROPTICS INC [US]; SIMINOU KAMRAN [US]) 12 November 2009 (2009-11-12) | 16,17 | INV. A61B3/11 A61B5/16 |
| A | * paragraphs [0002], [0019], [0022], [0031] * * figure 1 * | 1-15 | A61B5/00 G16H50/20 G16H50/70 |
| A | ONG C ET AL: "The Effect of Ambient Light Conditions on Quantitative Pupillometry", NEUROCRITICAL CARE, SPRINGER US, NEW YORK, vol. 30, no. 2, 14 September 2018 (2018-09-14), pages 316-321, XP036728172, ISSN: 1541-6933, DOI: 10.1007/S12028-018-0607-8 [retrieved on 2018-09-14] * abstract * | 1-17 | |
| A | HAWKES MAXIMILIANO A ET AL: "Neurological Prognostication After Cardiac Arrest in the Era of Target Temperature Management", CURRENT NEUROLOGY AND NEUROSCIENCE REPORTS, SPRINGER US, NEW YORK, vol. 19, no. 2, 9 February 2019 (2019-02-09), pages 1-8, XP036698160, ISSN: 1528-4042, DOI: 10.1007/S11910-019-0922-2 [retrieved on 2019-02-09] * abstract * * page 3, left-hand column, paragraph 3 * | 1-17 | **TECHNICAL FIELDS SEARCHED (IPC)** A61B G16H |

-/--

The present search report has been drawn up for all claims

| Place of search | Date of completion of the search | Examiner |
|---|---|---|
| Munich | 23 May 2024 | Gärtner, Andreas |

EPO FORM 1503 03.82 (P04C01)

page 1 of 2

|  | Europäisches<br>Patentamt<br>European<br>Patent Office<br>Office européen<br>des brevets | **EUROPEAN SEARCH REPORT** | Application Number<br>EP 23 46 1699 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | AGARWAL SACHIN ET AL: "The Influence of Therapeutics on Prognostication After Cardiac Arrest", CURRENT TREATMENT OPTIONS IN NEUROLOGY, SPRINGER US, NEW YORK, vol. 21, no. 12, 25 November 2019 (2019-11-25), XP036952507, ISSN: 1092-8480, DOI: 10.1007/S11940-019-0602-1 [retrieved on 2019-11-25] * abstract * * page 6, paragraph 4 *<br>----- | 1-17 | |

TECHNICAL FIELDS
SEARCHED      (IPC)

The present search report has been drawn up for all claims

| Place of search | Date of completion of the search | Examiner |
|---|---|---|
| Munich | 23 May 2024 | Gärtner, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 .................................................................................
& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 23 46 1699

This annex lists the patent family members relating to the patent documents cited in the above-mentioned European search report.
The members are as contained in the European Patent Office EDP file on
The European Patent Office is in no way liable for these particulars which are merely given for the purpose of information.

23-05-2024

| Patent document cited in search report | Publication date | Patent family member(s) | | Publication date |
|---|---|---|---|---|
| WO 2009137614 A2 | 12-11-2009 | US | 2009306538 A1 | 10-12-2009 |
| | | WO | 2009137614 A2 | 12-11-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009137614 A2
- WO 2023059325 A1
- US 20190159671 A
- US 9402542 B2
- WO 2010062400 A1
- EP 23461698

**Non-patent literature cited in the description**

- **GHAURI, MUHAMMAD S et al.** Evaluating the reliability of neurological pupillary index as a prognostic measurement of neurological function in critical care patients. *Cureus*, 2022, vol. 14 (9)
- **ONG C** ; **HUTCH M** ; **SMIRNAKIS S**. The Effect of Ambient Light Conditions on Quantitative Pupillometry. *Neurocrit Care.*, April 2019, vol. 30 (2), 316-321
- **W. TAYLOR et al.** Quantitative pupillometry, a new technology: normative data and preliminary observations in patients with acute head injury. Technical note. *JOURNAL OF NEUROSURGERY*, 2003, vol. 98 (1)